## Europäisches Patentamt
## European Patent Office
## Office européen des brevets

(19)

(11) Numéro de publication: **0 086 122**
**B1**

(12) **FASCICULE DE BREVET EUROPÉEN**

(45) Date de publication du fascicule du brevet:
18.03.87

(21) Numéro de dépôt: **83400095.2**

(22) Date de dépôt: **14.01.83**

(51) Int. Cl.⁴: **A 61 K  31/765,** A 61 K  31/52 //
(A61K31/765, 31:52, 31:10)

(54) Composition destinée à réaliser la reversion tumorale et son emploi en cancérologie.

(30) Priorité: **29.01.82  FR 8201397**

(43) Date de publication de la demande:
**17.08.83 Bulletin 83/33**

(45) Mention de la délivrance du brevet:
**18.03.87 Bulletin 87/12**

(84) Etats contractants désignés:
**BE CH DE GB IT LI LU NL SE**

(56) Documents cité:
**CA-A-824 650**
**GB-A-1 043 012**

**CHEMICAL ABSTRACTS, vol. 96, no. 14, 05 avril 1982, page 379, col.I, abrégé no. 110039j, Columbus, Ohio, US**

(73) Titulaire: **Bocquet, Roger Louis Eugène, 17 rue Chartran, F-92200 Neuilly sur Seine (FR)**

(72) Inventeur: **Bocquet, Roger Louis Eugène, 17 rue Chartran, F-92200 Neuilly sur Seine (FR)**

(74) Mandataire: **Bouju, André, Cabinet Bouju 38 avenue de la Grande Armée, F-75017 Paris (FR)**

EP 0 086 122 B1

LIBER, STOCKHOLM 1987

## Description

La présente invention concerne une composition destinée à réaliser la reversion tumorale et son emploi en cancérologie.

Jusqu'à présent il est flagrant que depuis la plus haute antiquité le cancer était considéré par la "médecine" comme l'apparition d'une néoformation parasite, indésirable, qu'il était donc indispensable d'éliminer pour en libérer l'organisme.

De tout temps la réaction médicale s'est matérialisée par un "réflexe primaire d'extirpation" en relation avec cette façon de voir les choses.

C'est cet état d'esprit qui a permis d'élaborer des techniques de destruction des cellules cancéreuses à commencer par le plus simple : l'éxerèse chirurgicale.

A l'heure actuelle et avec le progrès, elle demeure toujours valable, sinon efficace. En effet utilisée seule, trop souvent elle est incomplète, et trop souvent aussi, elle a pour conséquences de disséminer le mal.

Puis on a fait appel à la radiothérapie, sous forme de rayons X, radium, cesium, cobalt. Cette thérapeutique est basée sur le fait qu'elle détruit les cellules d'autant plus facilement qu'elles se multiplient plus activement comme c'est le cas pour les cellules cancéreuses qui se montrent par là plus fragiles et plus sensibles aux rayons.

Malheureusement aussi, les rayons facilitent l'apparition de nouvelles cellules cancéreuses à partir de cellules saines comme cela a été amplement observé à la suite de l'explosion atomique d'Hiroshima.

Enfin on a fondé beaucoup d'espoirs sur la chimie, et la production de drogues nouvelles du type antimitotiques. Les drogues antimitotiques agissent surtout au niveau de l'ADN et de son expression en inhibant le processus de replication. Malheureusement elles s'attaquent indifféremment aux cellules cancéreuses, et aux cellules normales en prolifération rapide, dont la reproduction est indispensable pour maintenir l'organisme entier en parfait état d'équilibre.

Par ailleurs l'éradication d'une tumeur par chimio thérapie, si elle suit un processus de réduction logarithmique très spectaculaire dans les premiers temps n'arrive jamais à la dernière cellule.

Enfin il est un écueil majeur qui vient d'être révélé : c'est que, agissant au niveau de l'ADN pour provoquer l'arrêt de sa reproduction en particulier, les drogues antimitotiques provoquent des mutations dont quelques-unes facilitent l'apparition d'un nouveau cancer quelques années plus tard.

C'est pourquoi, à l'heure actuelle, la thérapeutique par stimulation immunologique connaît un regain d'actualité, et se trouve activement étudiée.

Or il est une autre conception du problème, qui semblait impensable autrefois dans l'ignorance où nous étions (et sommes encore) des processus de cancérisation. Il s'agit de la REVERSION TUMORALE qui consiste à faire redevenir normales les cellules cancéreuses sans les tuer.

Cette solution est séduisante à tel point que le 13 Octobre 1981 lors de la remise du Prix Griffuel à l'UNESCO il a été déclaré que la Reversion Tumorale serait une "véritable révolution biologique".

Dans cette optique on réalise ce que l'on admet aujourd'hui à savoir que la cellule cancéreuse est une ancienne cellule normale, de l'organisme même où elle se trouve, donc non pathologique, mais déviée dans son comportement pour une raison que nous ignorons encore, mais qui aboutit à une invasion déterminant un état de déséquilibre et d'anarchie tel que la mort s'ensuit.

En conséquence, en lui permettant de retrouver l'expression normale d'une programmation établie, en équilibre avec l'ensemble de l'organisme, la cellule originellement normale, puis, "transformée" en cellule cancéreuse, retrouve à nouveau une activité normale.

Ce n'est pas d'hier que la "Recherche" s'est penchée sur le problème de la reversion tumorale, confortée en cela par le fait que certains, mais trop rares cancers, pouvaient regresser spontanément.

De nombreux auteurs ont essayé des drogues chimiques susceptibles d'avoir des propriétés revertantes, mais sans succès.

Ainsi, le GB-A-1 043 012 décrit une composition pharmaceutique renfermant un agent inhibiteur de la croissance des tumeurs, tel que le triéthylèneglycol diglycidyl éther associé avec un composé tel que le diméthylsulfoxyde qui renforce l'action inhibitrice de l'agent précité. Cependant, cette composition ne produit aucune reversion tumorale.

Or c'est le 27 février 1981 qu'il me fut donné, après plusieurs années d'études et d'expérimentation sur de nombreux revertants possibles, de réaliser, in vitro, la reversion des cellules HeLa en utilisant non "un" revertant, mais une composition revertante alliant les propriétés complémentaires de ses composants, évitant ainsi l'écueil de la drogue unique, impuissante par elle seule à réaliser la reversion tant recherchée.

La reversion tumorale s'est manifestée par la réapparition de deux caractères classiques et bien connus en matière de culture cellulaire, à savoir la réapparition de l'inhibition de contact, et la qualité de l'adhérence au verre.

Ce phénomène constitue bien une révolution car la reversion s'accompagne d'un abandon de la prolifération anarchique pour retrouver l'inhibition de contact, qui n'est autre que l'arrêt de la prolifération lorsque le tapis cellulaire en culture s'étend à toute la surface du flacon dont le fond lui sert de support.

Par ailleurs la normalisation des cellules originellement cancéreuses se matérialise aussi par une meilleure adhérence soit entre elles, soit au verre.

Il est bien connu que les cellules cancéreuses sont facilement détachables, et c'est là une raison majeure et favorisante de la dissémination métastatique.

La composition revertante non toxique aboutissant à ces deux caractères confirmés, il était logique d'en étudier l'intérêt dans le domaine thérapeutique.

Utilisable très facilement, elle inhibe effectivement la croissance tumorale pour laisser alors au reste de l'organisme le soin de se débarrasser par ses propres moyens (homéostatiques, mais surtout immunologiques) donc des moyens naturels, des cellules qui, de dangereuses qu'elles étaient sont devenues simplement inutiles.

Suivant l'invention, cette composition est caractérisée par l'association de trois produits chimiques : le polyéthylène glycol - le diméthylsulfoxyde et la théophylline réalisée par simple mélange dans de l'eau distillée.

Ajouté à une culture de cellules cancéreuses dans le milieu de culture même qui les baigne (cellules de la lignée HeLa par exemple) ce mélange fait apparaître la reversion tumorale.

La composition et le procédé d'utilisation de la composition seront décrits avec plus de précision selon l'exemple non limitatif suivant.

## Descriptif expérimental

Un tapis confluent de cellules HeLa (cellules cancéreuses) en culture a été traité par l'invention. Il s'agissait d'une culture en boîte Falcon de 75 cm$^2$ de surface contenant 30 ml de milieu de culture (MEM 2011 Eurobio) complété à 10 % de sérum de veau foetal (SVF Eurobio).

### 1è experience - Experience de base - Le Phéno -mène de la Réversion

Flacon n° 1. Il fut ajouté le mélange établi dans les proportions suivantes :

1 ml 5 d'une solution à 10 % de polyéthylène glycol

0 ml 5 de diméthylsulfoxyde pur

50 microlitres de théophylline

A la suite de quoi le milieu a été renouvelé deux fois à une semaine d'intervalle et dans les proportions décrites ci-dessus.

Enfin la culture a été renouvelée de semaine en semaine sans qu'il soit rajouté à nouveau la composition revertante.

Parallèlement à cette expérience de base d'autres flacons de même nature ont été mis en compétition.

Flacon n° 2. Flacon témoin sans aucune addition pour témoigner de l'évolution normale de la culture HeLa dans son milieu habituel.

Flacon n° 3. Additionné seulement de polyéthylène glycol (1,5 ml de la solution à 10 %).

Flacon n° 4. Additionné seulement de diméthylsulfoxyde pur (0,5 ml).

Flacon n° 5. Additionné seulement de 50 microlitres de theophylline.

Flacon n° 6. Additionné par contre de polyéthylène glycol (1,5 ml de la solution à 10 %) et de diméthylsulfoxyde pur (0,5 ml) sans théophylline.

## Résultats

Flacon n° 1. Huit jours après le temps 0 on pouvait déjà distinguer des cellules en disposition régulièrement ordonnée qui continuaient à rester collées au verre alors que dans d'autres endroits le caractère anarchique des cellules originelles persistait. Puis au fur et à mesure des renouvellements du milieu, on pouvait voir que le processus engagé se poursuivait pour arriver un mois à six semaines plus tard à obtenir un tapis cellulaire strictement monocouche et étendu à toute la surface du flacon après avoir réussi à éliminer les cellules non reversées, repoussées, décollées et véritablement sapées par les cellules normales.

Flacon n° 2. La culture de cellules HeLa s'est décollée et les cellules sont passées en suspension comme il est courant de l'observer dans ce type de culture et cela au bout de trois semaines.

Flacon n° 3. L'addition de polyéthylène glycol n'a rien changé à la culture qui a suivi l'évolution du flacon n° 2.

Flacon n° 4. L'addition de diméthylsulfoxyde n'a rien changé à la culture qui a suivi l'évolution des flacons n° 2 et 3.

Flacon n° 5. L'addition de théophylline n'a rien changé non plus à la culture qui a suivi l'évolution des flacons n° 2, n° 3 et n° 4.

Flacon n° 6. Dans ce flacon par contre il a pu être observé au bout de 8 jours des plages de cellules apparemment reversées, en disposition régulièrement ordonnée et qui continuaient à rester collées au verre au milieu du tapis apparemment anarchique.

Mais au fur et à mesure des renouvellements du milieu le processus engagé ne semblait pas se poursuivre. Six semaines plus tard l'aspect de la culture était resté dans le même état - état quiescent - sans obtenir un tapis étendu à toute la surface du flacon. Cependant il est à noter un caractère important : le maintien en place des cellules de la culture - alors que dans le flacon témoin pendant le même temps toutes les cellules étaient en suspension depuis déjà trois semaines.

Après six semaines, bien que l'expérience soit terminée, le flacon n° 1 a été conservé. Or dix huit mois après on constate toujours que la culture cellulaire reversée reste dans le même etat - nommé quiescent- monocouche, et limité aux bords du flacon.

### 2è expérience - Compétition entre cellules HeLa et cellules reversées.

Pendant l'expérimentation de base, des tapis cellulaires HeLa autres que le tapis HeLa témoin étaient entretenus simplement pour maintenir la souche en lignée continue.

Or il s'est produit une "contamination" accidentelle par des cellules reversées sur l'un de ces tapis. Il s'est alors produit un véritable ensemencement cellulaire (analogue à un ensemencement microbien) et des colonies éparses sont apparues dix a quinze jours après, comme un mouchetis.

Puis ces colonies se sont mises à prendre possession du fond du flacon en sapant la culture. Elles s'agrandirent en formant des cercles, des disques de culture cellulaire strictement monocouche, qui se rejoignirent et devinrent confluents, repoussant devant eux sur le tapis cellulaire HeLa en produisant au contact la formation d'une sorte de boursouflure, une sorte de lèvre.

Le tapis HeLa ne fut bientôt plus qu'un maillage (comme un grillage de fil de fer). Enfin détruites, dilacérées, désorganisées, les cellules HeLa passant en suspension les unes après les autres, il ne reste plus alors qu'un tissu de cellules reversées monocouche et limité aux bords du flacon.

Il ressort de cette expérience que les cellules reversées présentent une bien meilleure adhérence au verre et une vitalité supérieure aux cellules HeLa en culture (ce qui a été vérifié ultérieurement par le fait que leur trypsination est plus difficile à effectuer). A la suite de cette expérience réalisée sans avoir été projetée, le tapis des cellules reversées ayant colonisé tout le fond du flacon a observé l'inhibition de contact. Il est resté alors quiescent, état dans lequel il se maintient depuis seize mois.

### 3è expérience - Maintien de l'état quiescent.

L'un des flacons contenant des cellules reversées à l'état quiescent ayant été négligé pendant 4 semaines, 8 mois après le temps 0, certaines cellules reversées sont mortes, se sont décollées, sont passées en suspension laissant apparaître des trous dans le tapis cellulaire, le milieu de culture par ailleurs étant devenu acide.

Après rinçage et renouvellement le tapis s'est reconstitué intégralement et la culture a retrouvé son aspect quiescent qu'elle connaissait. A dix huit mois du temps 0 elle se trouvait toujours dans le même état.

### 4è expérience - Mise en évidence du caractère héréditaire de la réapparition de l'inhibition de contact.

Au temps 0 l'un des flacons contenant des cellules reversées à l'état quiescent a été trypsiné pour réaliser un premier "passage" (terme désignant une opération identique au repiquage bactériologique).

La culture constitue sans difficulté un nouveau tapis monocouche qui devint quiescent dès qu'il eut atteint les bords du flacon.

Un 2è passage fut réalisé à 23 jours (temps j. 23)

Un 3è passage fut réalisé à 26 jours après (temps j. 49)

Un 4è passage fut réalisé à 25 jours après (temps j. 74)

Enfin un 5è et dernier passage fut réalisé 16 jours après (temps j. 90)

Au bout de cinq passages, l'expérience s'étant déroulée sur 90 jours et les conditions expérimentales démontrant amplement une constance dans la reproduction du phénomène, l'expérience fut arrêtée en considérant comme prouvés l'etablissement en lignée continue et le caractère héréditaire des cellules HeLa reversées. Depuis lors il est possible d'utiliser en permanence ces cellules reversées comme s'agissant des cellules d'une lignée continue nouvelle pour d'autres besoins du laboratoire.

### 5è expérience - Mise en évidence de l'indépen dance de la reprise de la culture (à partir de l'état quiescent) vis-à-vis du temps.

Pour que cette dernière expérience soit démonstrative il fut fait appel à un flacon de cellules reversées en culture quiescente et âgée de dix huit mois. Après trypsination ces cellules ensemencées ont colonisé le nouveau flacon. Une suite de trois passages réussie a même été effectuée à titre de confirmation.

La démonstration expérimentale de la reversion tumorale était faite. Elle était due ni au hasard ni à un artefact, et de plus la reproductibilité était un fait acquis.

En résumé, les expériences ci-dessus décrites demontrent que :
- La composition revertante réalise, sur les cellules HeLa en culture la reversion tumorale.
- Cette reversion se caractérise par la réapparition de caractères essentiels que la transformation cancéreuse avait fait disparaitre, et au nombre desquels ont été identifiées : l'inhibition de contact, une meilleure adhérence au verre et une meilleure cohésion entre les cellules constitutives du tissu cellulaire.
- Il a été mis en évidence que l'inhibition de contact en particuiier se conservait au fur et à mesure des mitoses nouvelles, que ce caractère était donc héréditairement transmis au sein des générations successives et qu'il était indépendant du temps de reprise de la culture et de la longueur des périodes de quiescence.

- Il a été mis en évidence qu'il était indispensable d'associer au moins deux composants à savoir le polyéthylène glycol et le diméthylsulfoxyde - mais aussi d'y ajouter la théophylline qui donne toute sa valeur a la reversion.

- Donc l'action revertante de la composition repose sur l'association d'un modificateur membranaire le polyéthylène glycol (dont le poids moleculaire de 1 000 à 6 000 est indifférent) et d'un revertant le diméthylsulfoxyde auxquels est ajouté un adjuvant, la théophylline.

- Les proportions indiquées dans la première experience constituent une base et une moyenne.

Des expériences complémentaires ont permis de mettre en évidence que :

1. La proportion du modificateur membranaire (polyéthylène glycol) atoxique ne peut être affecté que par les limites de sa viscosité, sa proportion optimum se situant à 15 % ( $\pm$ 7 %) en solution dans de l'eau distillée.

2. La proportion du revertant de base (diméthylsulfoxyde) quasiment atoxique peut être accrue de façon importante. Ce composé ne connait pas de limite de viscosité car sa vitesse de diffusion est très connue pour être incroyablement grande. Donc si l'on peut facilement la fixer à 20 % ( $\pm$ 10 %) du volume de la solution, une augmentation de la proportion n'est pas en elle-même un avantage à rechercher systématiquement.

3. La proportion de la théophylline par contre devra observer une fourchette assez étroite. La théophylline fixée à 50 microlitres pour 30 ml de milieu est une norme expérimentale de laquelle il est prudent de ne pas trop s'écarter en observant donc une proportion de 5 % ($\pm$ 2,5 %) de la solution.

**Extension de l'application de l'invention du domaine in vitro au domaine in vivo.**

Après les résultats obtenus et vérifiés in vitro, il est logique de penser à une utilisation dans le domaine de la thérapeutique en qualité de médicament anticancéreux.

Pour cela il faut s'appuyer sur deux séries d'informations :

1°) Informations toxicologiques

2°) Expérimentation clinique

Afin d'étayer les possibilités d'utilisation industrielle pharmaceutique de l'invention, ces deux points vont être successivement abordés.

1°) Informations toxicologiques

a) Le polyéthylène glycol est un composé chimique neutre dépourvu de toute toxicité et qui, déjà utilisé en injections sous-cutanées ou intraveineuses, n'a jamais eu aucun effet immédiat (allergique) ou secondaire, que ce soit sur l'animal ou chez l'homme.

b) Le diméthylsulfoxyde $C_2H_6OS$ a fait l'objet d'études cliniques très approfondies en même temps que très variées - autant chez l'homme que sur l'animal à la suite d'études toxicologiques sur un certain nombre d'espèces animales et sur l'homme.

Toutes ces recherches et études ont fait l'objet d'un compte rendu de 671 pages publié le 15 Mars 1967 dans les Annales de l'Académie des Sciences de New York (vol. n° 141, art. 1, p. 1 - 671). Mais à aucun moment ce composé chimique n'est cité comme revertant.

Par ailleurs, il ressort de ces études que le diméthylsulfoxyde est dépourvu de toxicité.

c) La théophylline $C_7H_8N_4O_2$ (diméthyl- 1,3 xanthine) est un composé chimique utilisé comme médicament depuis de très nombreuses années, diurétique, tonicardiaque et vasodilatateur bronchique employé en particulier en cas de crises d'asthme.

Il est donc inutile de reprendre in extenso une étude toxicologique nouvelle de ces trois composants.

Cependant on peut citer les DL50 suivantes :.

- Polyéthylène glycol

Chez le rat la DL50 du polyéthylène glycol (600) est 7,8 gramme/kilog. ce qui rapporté à l'homme (de 70 kg environ) ferait une dose de 546 grammes, alors que la dose jugée suffisante est seulement le 1/546 ème de cette DL50.

- Diméthylsulfoxyde

Chez la souris la DL50 du diméthylsulfoxyde est 12 grammes/kilog. et chez le rat 20,5 grammes /kilog. C'est une toxicité systémique basse qui produit les mêmes manifestations sur toutes les espèces. En comparaison la dose utilisée chez l'homme en une seule injection est le 1/1050 ème de cette DL50.

- Théophylline

Chez la souris la DL50 de la théophylline est 400 mg/kilog. et chez le rat 300 mg/kilog. Ces valeurs sont importantes.

Si par comparaison on les rapporte à la dose thérapeutique maximum utilisée chez l'homme, c'est-à-dire 0,02 gramme de théophylline pure pour une seule injection de 10 ml de produit, cette dose n'est que le 1/1225 ème de la DL50 moyenne de 350 mg/kilog.

2°) Expérimentation clinique

Très sommaire pour l'instant elle permet pourtant de retenir les observations importantes suivantes obtenues après une série d'injections par voie souscutanée espacées en principe de 8 jours à raison de 10 ml à chaque injection du mélange suivant :

Eau distillée : 90 ml

Polyéthylène glycol : 9 grammes

Diméthylsulfoxyde pur : 9 ml

Théophylline : 3 ml de la solution pharmaceutique courante à 6 %.

a) Sur le chien

Il a été réalisé : - l'obtention d'un flétrissement, d'un dessèchement et la disparition à 90 % d'une néoformation épidermique (petite tumeur blanche secondaire, à peau lisse et sans poils) après 3 injections espacées exceptionnellement de 4 à 5 jours seulement.

- la transformation sur le même animal de la tumeur principale : un sarcome de la face sous oculaire, volumineux qui est devenu fluctuant pour en arriver à la fonte purulente et à l'écoulement externe comme un abcès froid, après 10 injections espacées de 8 jours à partir de la troisième.

b) Sur l'homme

Il a été obtenu sur un leucémique

- à quelques jours de l'issue fatale - la chute du nombre des éléments de la lignée blanche de 100.000 à 10.000 en 48 heures avec conservation de la valeur fonctionnelle des éléments restants à la suite de 2 injections seulement de 10 ml effectuées à 24 heures d'intervalle. Cette valeur fonctionnelle a été matérialisée par un abaissement des blastes de 92 à 33 et le relèvement des lymphocytes de 1 à 65. Quelques jours encore après, les blastes étaient descendus à 7 et les lymphocytes en progression jusqu'à 87.

c) Sur la femme

- Un cancer du sein - carcinome de 7 mm de diamètre contrôlé par radiographie et échographie (la biopsie scientifiquement souhaitable ayant été rejetée pour ne pas faire courir à la malade un risque inutile) a été reversé après 5 injections de la composition revertante (injections sous-cutanées de 10 ml espacées de 8 jours). Ce traitement a été entretenu ensuite sur le rythme d'une injection tous les 10 jours pendant 2 mois après obtention de la reversion.

Cette reversion a été suivie par des radiographies périodiques prises tous les mois et par une échographie qui révéle que "l'image tumorale elle-même s'est modifiée et qu'il existe maintenant une zone hypoéchogène centrale avec renforcement d'échos postérieurs légèrement plus en dehors".

- Un cancer de l'utérus a été constaté par biopsie cervicale "Très important épidermoïde différencié mature invasif infiltrant exocervical".

Le traitement a consisté en radiothérapie locale au cesium. Puis une laparotomie a été pratiquée, non suivie d'exérèse, mais elle fut l'occasion d'une nouvelle biopsie - celle du ganglion de la bifurcation iliaque qui présentait à l'analyse "une infiltration massive par de nombreux boyaux néoplasiques épidermoïdes peu différenciés et immatures".

Le compte-rendu opératoire enfin fait état d'une tumeur inopérable du fait de sa situation à la fois vésicale et utérine. Intervient alors un traitement de 5 injections de la composition revertante (10 ml en injections sous cutanées espacées de 8 jours). Traitement entretenu lui aussi et sur le même rythme que dans l'observation précédente.

A la suite de quoi une scannographie utérine decouvre que "l'utérus est augmenté de volume mais avec bords réguliers d'échostructure homogène".

Le compte-rendu précise "qu'il n'existe pas de masse pelvienne actuellement individualisable".

Pour terminer, un frottis vaginal conclut à l'absence de toute malignité.

**En conclusion:**

- L'invention après avoir présenté les caractéristiques d'une composition revertante in vitro conserve donc indéniablement ces mêmes caractéristiques in vivo.

- L'effet n'est pas celui d'un destructeur des cellules cancéreuses.

- La composition ne détruit d'ailleurs aucune cellule.

- Mais les cellules une fois reversées restent in situ et continuent à signaler leur présence radiographiquement ou échographiquement.

- Quoi qu'il en soit la démonstration est faite sur quelques vingt cas déjà, que la croissance cellulaire est toujours arrêtée.

- Il appartient alors à l'organisme lui-même d'éliminer par ses propres moyens (si ces moyens sont intacts) et au premier rang desquels il faut citer les défenses immunologiques, des cellules, qui, si elles ne présentent plus le danger de prolifération anarchique des cellules cancéreuses, doivent néanmoins être éradiquées, comme des cellules étrangères par un processus homéostatique (processus d'attaque lymphocytaire, de nécrose, ou de fonte purulente aseptique).

En l'état actuel de l'expérience acquise il faut envisager d'utiliser la composition revertante telle qu'elle a été définie et telle qu'elle a déjà été utilisée en thérapeutique.

Néanmoins il s'agit d'adapter un modèle élaboré pour une expérimentation in vitro au domaine in vivo.

Pour cela on remplace l'eau distillée par du sérum physiologique - ou par du sérum de Quinton.

Par ailleurs s'il semble bien établi que les fourchettes de dilution du Polyéthylène glycol et du Diméthylsulfoxyde sont efficaces il faut par contre adapter l'organisme à l'action de la Théophylline (Théophylline connue dans sa présentation pharmaceutique habituelle en solution à 6 %) en pratiquant une dilution d'approche pour les premières injections avec des doses réduites au 1/50è et au 1/100è de celles déjà définies et qui seront pratiquées ultérieurement.

**Revendications**

1. Composition destinée à réaliser la reversion tumorale et caractérisée en ce qu'elle comprend : du polyéthylène glycol (modificateur membranaire), du diméthylsulfoxyde (composé revertant de base) et de la théophylline (adjuvant).

2. Composition selon la revendication 1, caractérisée en ce qu'elle comprend : du polyéthylène glycol de poids moléculaire compris entre 1 000 et 6 000 en solution à 15% ( ± 7 %) dans de l'eau distillée, du diméthylsulfoxyde pur ajouté à la solution de polyéthylène glycol dans la proportion de 20% ( ± 10%), et de la théophylline ajoutée au mélange déjà obtenu dans la proportion de 5 % ( ± 2,5 %).

3. Médicament destiné à la reversion tumorale, caractérisé en ce qu'il comprend comme principes actifs l'association définie à la revendication 1.

**Patentansprüche**

1. Zusammensetzung zur Rückumwandlung von Tumorzellen, dadurch gekennzeichnet, daß sie Polyäthylenglykol (membranmodifizierendes Mittel), Dimethylsulfoxid (Grundmittel für die Rückumwandlung) und Theophyllin (Adjuvans) enthält.

2. Zusammensetzung nach Anspruch 1, dadurch gekennzeichnet, daß sie Polyäthylenglykol mit einem Molekulargewicht zwischen 1000 und 6000 in 15( ± 7)%iger Lösung in destilliertem Wasser, reines Dimethylsulfoxid, das der Polyäthylenglykol-Lösung zu einem Anteil von 20 % ( ± 10 %) zugefügt worden ist, und Theophyllin enthält, das dem erhaltenen Gemisch zu einem Anteil von 5 % ( ± 2,5 %) zugefügt wurde.

3. Arzneimittel zur Rückumwandlung von Tumorzellen, dadurch gekennzeichnet, daß es als Wirkstoffe die Stoffkombination gemäß Anspruch 1 enthält.

**Claims**

1. A composition for producing tumor reversion, characterized in that it comprises : polyethylene glycol (membrane modifier), dimethyl sulfoxide (a basic revertant compound) and theophylline (adjuvant).

2. A composition according to claim 1, wherein said composition comprises : polyethylene glycol having a molecular weight within the range of 1000 to 6000 in a 15% ( ± 7%) solution in distilled water, pure dimethyl sulfoxide added to the polyethylene glycol solution in a proportion of 20% ( ± 10%), and theophylline added to the mixture already obtained in a proportion of 5% ( ± 2.5%).

3. A drug for tumor reversion, wherein the active principles of said drug are provided by the association defined in claim 1.